# EUROPEAN PATENT APPLICATION

(11) **EP 4 311 579 A1**
(43) Date of publication of application: **31.01.2024**
(21) Application number: 22187784.8
(22) Date of filing: 29.07.2022
(51) Int. Cl.: A61P 37/06, C07K 16/28, C12N 15/113

(54) **B CELL-SPECIFIC MAB-SIRNA CONJUGATES IMPROVE MYASTHENIA**

(71) Applicant: Association Française contre les Myopathies, 75651 Paris Cedex 13 (FR); The Board of Regents of the University of Texas System, Galveston, TX 77555 (US)
(72) Inventor: HUDA, Ruksana, Texas, 77573 (US)
(74) Representative: Axe PI

(57) **Abstract**

The present invention relates to a combination of two antibody-RNA conjugates comprising an anti-BAFF receptor antibody or binding fragment thereof that is bound or conjugated to a small RNA that targets a BAFF receptor mRNA, and an anti- BCMA specific antibody or binding fragment thereof that is bound or conjugated to a small RNA that targets a BCMA mRNA. The composition comprising the combination will be used for the treatment of autoimmune disorders, preferably *Myasthenia Gravis.*

## Description

### FIELD OF THE INVENTION

The present invention relates to a combination of two antibody-RNA complexes, a composition comprising said combination, and a composition for use as a medicament, notably for the treatment of autoimmune muscle disorders, notably *Myasthenia gravis.*

### BACKGROUND

Myasthenia gravis (MG) is a chronic autoimmune neuromuscular disorder. It is characterized by muscle fatigue and weakness caused by antibody- and complement-mediated damage to the neuromuscular junction.

Patients with MG who are seropositive for autoantibodies to the acetylcholine receptor (AChR), muscle specific tyrosine (MuSK), or low-density lipoprotein receptor-related protein 4 (LRP4) present with voluntary muscle weakness due to dysfunctional neuromuscular junctions and impaired neuromuscular transmission. 70% of patients with MG exhibit seropositivity for AChR-specific autoantibodies and significantly reduced muscle function. The clinical signs of muscle fatigue include ptosis, diplopia, slurred speech, appendage tremor, and even respiratory failure due to disease worsening.

Thus far, approximately 700,000 cases of MG with either generalized or ocular pathology have been reported worldwide.

There is a growing emphasis on developing new therapies for MG. Besides the traditional treatments, such as thymectomy, intravenous immunoglobulin, plasmapheresis, or corticosteroid, new treatments are needed for MG patients who do not attain disease remission by conventional treatments. In recent years, new non-immunosuppressive therapies for MG have focused mainly on target-specific therapies, particularly monoclonal antibody (mAb)-based therapies, such as rituximab, belimumab, and other neonatal Fc receptor-targeting mAbs. Several mAbs have been developed against the B cell-specific cluster of differentiation biomarker proteins to control B-cell maturation, differentiation, survival, and pathogenic autoantibody production. The primary goal of these treatments is to prevent pathogenic autoantibody production and complement activation. Reduction in circulatory levels of autoantibodies will block the AChR depletion as well as complement activation at the postsynaptic neuromuscular junction and, thus, will preserve muscle function.

The first therapeutic mAb for targeted therapy in patients was a mouse anti-CD3 mAb, muromonab, used to prevent tissue rejection. Subsequently, mAbs have been engineered to incorporate both human and mouse sequences (humanized), followed by production of fully human recombinant mAbs.

Rituximab or RTX (also known as Rituxan, Rixathon, or Truxima [Genentech, San Francisco, CA, USA]) is a chimeric murine-human IgG1 k mAb that targets CD20, a 33-kDa protein expressed on pro-B cells and all mature B cells, but not long-lived plasma or plasmablast cells. CD20 has an important role in the growth and differentiation of B cells into plasma cells, and rituximab can efficiently deplete CD20-positive B cells in MG patients. However, it is ineffective in reducing pathogenic AChR-Ab levels. Long-lived plasma cells are the major producers of autoAb and lack CD20, hence rituximab likely targets only short-lived plasma cells and CD20+, IL10-producing B-regs, or B10 cells, and reduction of autoAb is generally short term and insufficient, resulting in only transient clinical improvement. Thus, rituximab-treated AChR-MG and MuSK-MG patients often have disease relapse or recurrence after an initial phase of disease peptides from the IgG immune complexes.

Hence, there remains a need for always more efficient treatments that will reduce pathogenic AChR-Ab levels.

### PROBLEM TO BE SOLVED

The technical problem underlying the present invention is to prevent pathogenic autoantibody production and complement activation. Reduction in circulatory levels of autoantibodies will block the AChR depletion as well as complement activation at the postsynaptic neuromuscular junction and, thus, will preserve muscle function.

The technical problem is solved by the embodiments provided herein below and as characterized in the appended claims.

### SUMMARY OF THE INVENTION

In a first aspect, the invention relates to a combination of two antibody-RNA complexes comprising:
- an anti-BAFF receptor antibody or binding fragment thereof that is bound or conjugated to a small RNA that targets a BAFF receptor mRNA, and
- an anti-BCMA specific receptor antibody or binding fragment thereof that is bound or conjugated to a small RNA that targets a BCMA receptor mRNA.

In another aspect, the invention relates to a composition comprising the combination according to the invention.

In a further aspect, the invention relates to the composition according to the invention, for use as a medicament.

In a particular aspect, the invention relates to the composition for use according to the invention, for the treatment of autoimmune disorders.

In a more particular aspect, the invention relates to the composition for use according to the invention, for the treatment of autoimmune muscle disorders, preferably *Myasthenia gravis.*

The Applicant has been able to demonstrate that the duoconjugate has a potential therapeutic value for the treatment of MG and other autoimmune diseases.

The Applicant has also been able to observe that PEGylation further improves the efficacy of duoconjugate treatment in terms of a higher reduction in the level of autoantibodies and clinical improvement in the mouse model of *Myasthenia gravis.*

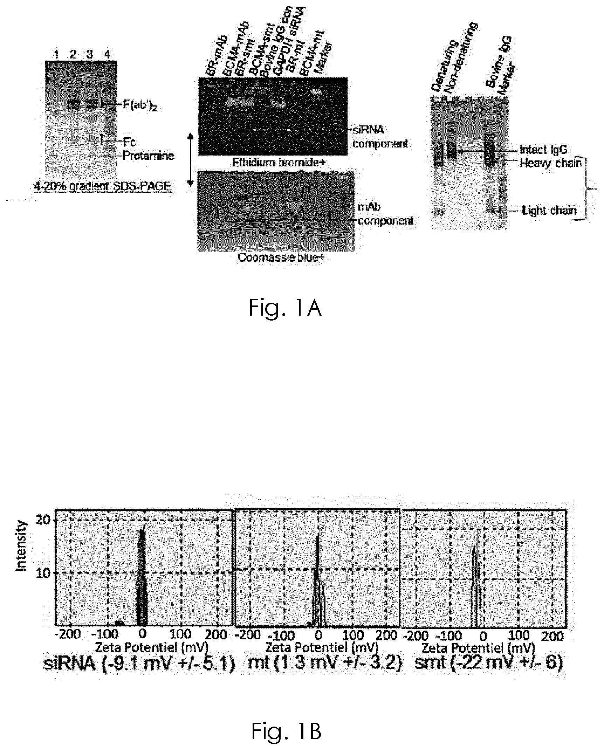

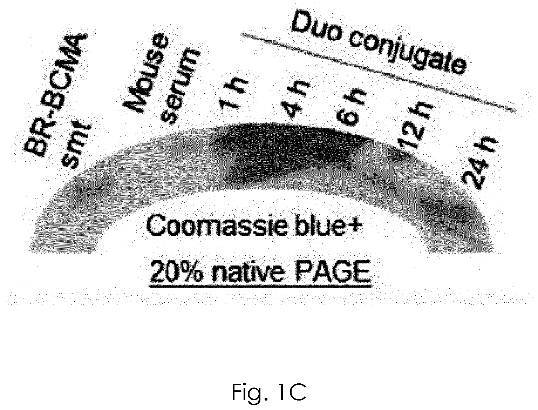

Further aspects and advantages of the present invention are described in the following description (with reference to Figures 1 to 6), which should be regarded as illustrative and not limiting the scope of the present application.

### BRIEF DESCRIPTION OF THE DRAWINGS

Figure 1 shows the preparation of mAb/siRNA conjugates.
**1A)** Purification of protaminated mAbs.
   [Top left] Purification of protaminated mAbs. Protamine alone (lane 1) and contaminating unreacted or free protamine removed (or not) from protamine-linked mAbs through gel filtration (lanes 2 and 3, respectively).
   [Top middle] Verification of the presence of mAb and siRNA in the conjugates (representative gel image). The conjugates with appropriate controls were electrophoresed in a native polyacrylamide gel. The strong association of siRNA with the mAb alters the electrophoretic mobility of the mAb and both siRNAs. Lanes 3 and 4 (top panel) show the siRNA components of the conjugates detected via staining of the gel with ethidium bromide (top panel). The mAb components were detected by restaining the gel with coomassie brilliant blue (lower panel).
   [Top right] Conjugates eluted from a 10% native polyacrylamide gel were subsequently resolved in a 4%-20% sodium dodecyl sulfate-polyacrylamide gel. Gel-eluted conjugate bands with reducing and nonreducing loading dye and bovine IgG control (lanes 1, 2, and 5, respectively).
**1B)** Zeta potential (surface charge) measurements of siRNAs, protaminated mAb (mt), and duoconjugate (smt).
**1C)** Serum stability of the conjugates.

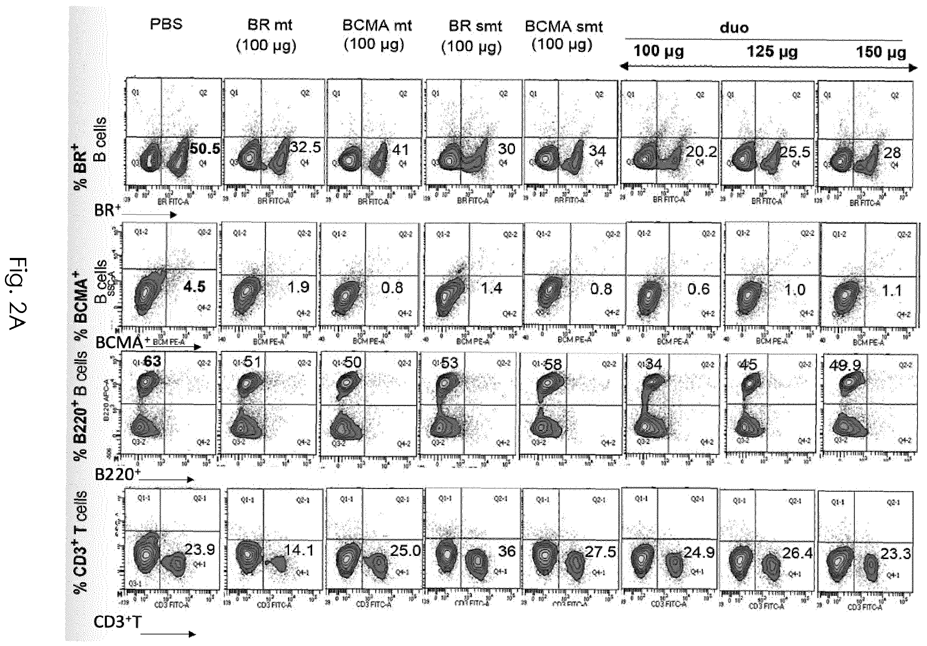

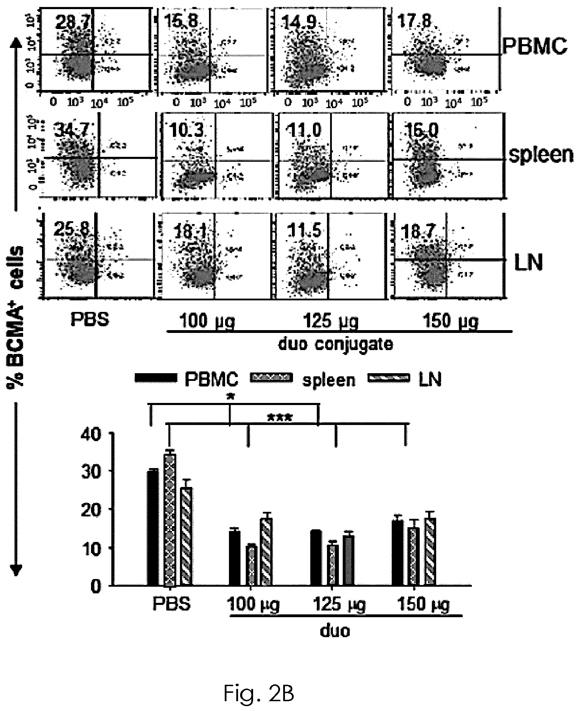

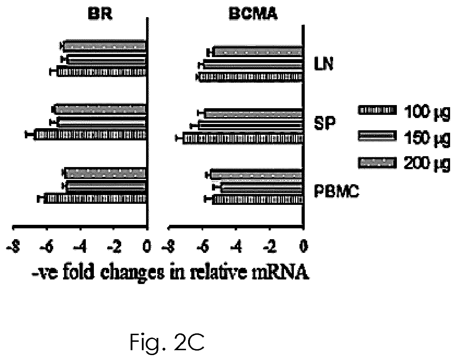

   Figure 2 shows the dose kinetics: conjugate-mediated suppression of target receptor-expressing B cells.
**2A)** Flow analysis of isolated splenocytes (3 dpt) stained with fluorochrome-conjugated antibodies specific for B cells expressing CD268, CD269, and B220 on their surface (shown in rows).
**2B)** BCMA+ cell frequencies in the spleen, lymph nodes, and PBMCs (3 dpt) with three doses of mAb-siRNA conjugates, also shown in bar chart (bottom).
**2C)** Relative expression of BR and BCMA normalized to beta-actin via qRT-PCR.

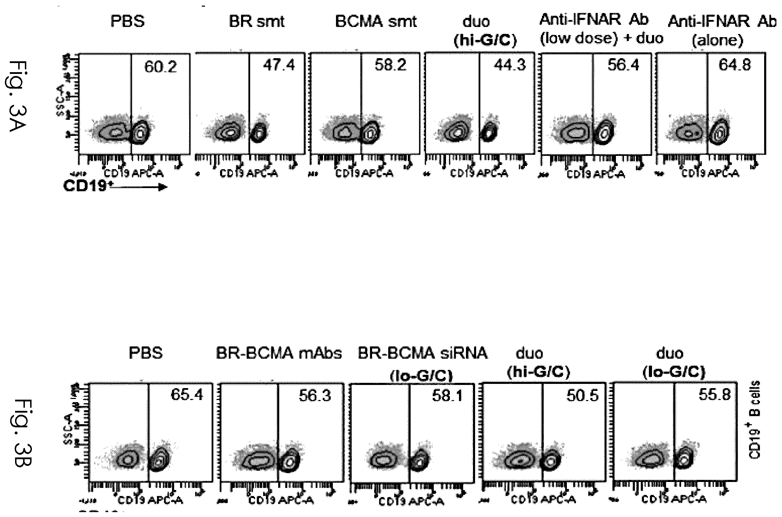

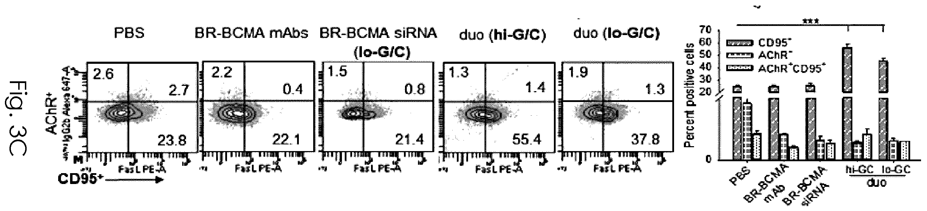

   Figure 3 shows CD19+ cell frequencies following IFNAR neutralization, or siRNA GC content alterations in the conjugates (6wpt).
**3A)** Flow cytometric analysis of LN cells showing frequencies of CD19+ B cells of EAMG mice pretreated (or not) with IFNAR antibody before conjugate treatment or treated only with anti-IFNAR antibodies (6wpt).
**3B)** Frequencies of CD19+ cells after treatment of mice with siRNAs and duoconjugates comprising siRNAs with a high or low GC content (6wpt).
**3C)** Effect of GC content of siRNA on the frequencies of AChR+ B cells and CD95+ cells (6wpt).
   Figure 4 shows frequencies of total CD27+ memory cells.
**4A, 4B, 4C)** Frequencies of total CD27+, B220+, and CD4+ cells following treatment with conjugates (3 mpt).
   Figure 5 shows anti-AChR antibody affinity/levels in mice treated with the conjugates.
**5A, 5B)** Measurement of serum levels of pathogenic anti-AChR IgG2b by ELISA and a new serum antibody immunoprecipitation assay.
   Figure 6 shows
**6A)** grip strength of EAMG mice in treated and control groups.
**6B)** ELISA results for functional muscle AChR at 4 mpt, the experimental endpoint.

### DETAILED DESCRIPTION

In this description, unless it is specified otherwise, it is understood that, when an interval is given, it includes the upper and lower bounds of said interval.

The present invention provides materials and methods useful in therapy, more particularly for the treatment of autoimmune diseases. More specifically, the present invention provides a combination of mAbs and siRNAs that could potentially inhibit the functions of all pathogenic autoantibody-producing B cells and their precursor cells.

The advantages of the invention are more particularly shown with respect to the treatment of *Myasthenia gravis.*

The invention relates, in a first aspect, to a combination of two antibody-RNA complexes.

The term "antibody", "immunoglobulin", and "antibody/immunoglobulin binding fragments" as used herein refers to a polypeptide, more precisely, to polyclonal and monoclonal antibody preparations, as well as preparations including hybrid antibodies, altered antibodies , F(ab')2 fragments, F(ab) fragments, Fv fragments, single domain antibodies, chimeric antibodies, humanized antibodies, and functional fragments or a protein thereof that exhibit immunological binding properties of the antibody molecule against a specific antigenic target.

The term "polypeptide(s)" as used herein refers to any peptide or protein comprising two or more amino acids joined to each other by peptide bonds or modified peptide bonds. "Polypeptide(s)" refers to both short chains, commonly referred to as peptides, oligopeptides, and oligomers and to longer chains generally referred to as proteins. Polypeptides can contain amino acids other than the gene encoded amino acids. "Polypeptide(s)" include those modified either by natural processes, such as processing and other post-translational modifications.

As used herein, the term "antigen - binding site" or "binding portion" refers to the part of the antibody or immunoglobulin molecule that participates in specific antigen binding. The antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as" framework regions," or "FRs". Thus, the term "FR" refers to amino acid sequences that are naturally found between and adjacent to hypervariable regions in immunoglobulins. In an antibody molecule, the three hypervariable regions of a light chain and the three hypervariable regions of a heavy chain are disposed relative to each other in three-dimensional space to form an antigen - binding surface. The antigen-binding surface is complementary to the three - dimensional surface of a bound antigen, and the three hypervariable regions of each of the heavy and light chains are referred to as "complementarity - determining regions," or "CDRs."

The term "complex" as used herein refers preferably to the union of two molecules that are held together by forces that are chemical.

In a preferred embodiment, the antibody is a monoclonal antibody (mAb). As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')2, Fv, and other fragments that exhibit immunological binding properties of the parent monoclonal antibody molecule.

The combination according to the invention first comprises an anti-BAFF receptor antibody or binding fragment thereof that is bound or conjugated to a small RNA that targets a BAFF receptor mRNA. The anti-BAFF receptor antibody or binding fragment thereof that is bound or conjugated to a small RNA will degrade the BAFF receptor mRNAs.

As used herein, the term "BAFF receptor" refers to BAFF receptor (B - cell activating factor receptor, BAFF-R, BR), also known as tumor necrosis factor receptor superfamily member 13C (TNFRSF13C), which is a membrane protein of the TNF receptor superfamily, which recognizes BAFF. In humans, the BAFF receptor is encoded by the TNFRSF13C gene (UniProt Q96RJ3, GenBank AF373846.1); relevant sequences incorporated herein by reference.

The term "conjugated" as used herein refers to a compound formed by the joining of two or more chemical/biological compounds.

The combination according to the invention secondly comprises an anti-BCMA specific receptor antibody or binding fragment thereof that is bound or conjugated to a small RNA that targets a BCMA receptor mRNA. The anti-BCMA specific receptor antibody or binding fragment thereof that is bound or conjugated to a small RNA will degrade the BCMA receptor mRNAs.

As used herein "BCMA specific receptor" refers to BCMA receptor (B-cell maturation antigen receptor, BCMA-R), also known as tumor necrosis factor receptor superfamily member 17 (TNFRSF17), which is a membrane protein of the TNF receptor superfamily, which recognizes BCMA. In humans, the BCMA receptor is encoded by TNFRSF17 gene (UniProt Q02223, GenBank Z14954.1); relevant sequences incorporated herein by reference.

As used herein, the term "RNA" refers to Ribonucleic acid, which is a polynucleotide molecule essential in various biological roles in coding, decoding, regulation and expression of genes. RNA are nucleic acids.

The term "polynucleotide(s)" as used herein generally refers to a plurality, two or more, of "nucleotide(s)", *i.e*. any polyribonucleotide or polydeoxyribonucleotide, which may be unmodified RNA or DNA or modified RNA or DNA. Polynucleotide(s) include, without limitation, single- and double-stranded DNA, DNA that is a mixture of single- and double-stranded regions or single- and triple stranded regions, single- and double-stranded RNA, and RNA that is a mixture of single and double-stranded regions, hybrid molecules comprising DNA and RNA that can be single-stranded or, more typically, double-stranded, or triple-stranded regions, or a mixture of single- and double-stranded regions. As used herein, the term "polynucleotide(s)" also includes DNAs or RNAs as described above that contain one or more modified bases. Thus, DNAs or RNAs with backbones modified for stability or for other reasons are "polynucleotide(s)" as the term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as used herein embraces such chemically, enzymatically, or metabolically modified forms of polynucleotides, as well as the chemical forms of DNA and RNA characteristic of viruses and cells, including, for example, simple and complex cells. "Polynucleotide(s)" also embraces short polynucleotides often referred to as oligonucleotide(s).

"Small RNAs" or "small molecular weight RNAs" means non-coding silencer RNAs (ribonucleic acids) of small molecular weight, with a maximum length of 150 nucleotides, such as all types of small, single- and/or double-stranded non-messenger RNAs. They can inhibit the expression of target genes via post-transcriptional gene silencing and chromatin-dependent gene silencing, or nuclease mediated degradation in both the cytoplasm and the nucleus.

In the combination according to the present invention, the small RNAs are preferably miRNA, shRNA, siRNA, antisense RNA, and/or specific mRNA-targeting base/nucleotide sequence(s), more preferably siRNA.

As used herein, the term "miRNAs" refers to microRNAs. They inhibit the expression of multiple mRNAs. Indeed, miRNA is partially complementary to its target mRNA.

As used herein, the term "siRNAs" refers to small interfering RNAs. They inhibit the expression of one specific target mRNA. Indeed, siRNA is usually fully complementary to the coding region of its target mRNA. Alternatively, short hairpin RNAs (shRNAs) can be used to achieve a specific gene silencing effect via the RNAi mechanism. shRNAs are stem-loop RNAs, which are expressed in the nucleus, typically through the delivery of viral vectors. Once expressed, they are transported to the cytoplasm for further processing, and subsequently loaded into the RISC for specific gene silencing activity in the same manner as synthetic siRNAs.

The small RNAs that are used in the present invention are antisense silencer (synthesized *in vitro*) RNAs. Antisense RNA, also referred to as antisense transcript, natural antisense transcript or antisense oligonucleotide, is a single stranded RNA that is complementary to a protein coding messenger RNA with which it hybridizes, and thereby blocks its translation into protein.

In a preferred embodiment, the combination of two antibody-RNA complexes comprises:
- an anti-BAFF receptor antibody or binding fragment thereof that is bound or conjugated to an siRNA, shRNA, or both, that targets a BAFF receptor mRNA, and
- an anti-BCMA specific receptor antibody or binding fragment thereof that is bound or conjugated to an siRNA, shRNA, or both, that targets a BCMA receptor mRNA.

In a more preferred embodiment, the combination of two antibody-RNA complexes comprises:
- an anti-BAFF receptor antibody or binding fragment thereof that is bound or conjugated to an siRNA that targets a BAFF receptor mRNA, and
- an anti-BCMA specific receptor antibody or binding fragment thereof that is bound or conjugated to an siRNA that targets a BCMA receptor mRNA.

Preferably, the combination according to the present invention, comprises:
- the anti-BAFF receptor antibody and the siRNA, and
- the anti-BCMA-specific receptor antibody and the siRNA,
in which antibody and siRNA are electrostatically bound via small protein chemically cross-linked to the antibody with a linker.

The linker is associated with a small protein that electrostatically binds with the siRNA.

Electrostatic interactions are forces between nearby atoms and molecules. Like charges will repel one another and opposite charges will attract.

The term "Small proteins" or "small molecular weight protein" as used herein means polypetides that have a maximum length of a 100 amino acids.

In a preferred embodiment, the linker is a conditionally self-cleaving RNA sequence, a pH sensitive linker, a hydrophobic sensitive linker, a cleavable linker, a linker that provides a sorting signal, a linker that reduces steric hindrance, a linker that contributes to a condensing ability of the nucleic acid binding domain, a peptide or protein linker, a protamine linker, a polyK linker, or an HIV-TaT protein translocation (TPTV) linker.

In a more preferred embodiment, the linker is selected from glutaraldehyde, bissul fosuccinimidyl suberate, carbodiimide, bis ( succinimidyl ) penta ( ethylene glycol), bis ( succinimidyl) nona ( ethylene glycol), bis ( sulfosuccinimidyl) suberate, dimethyl suberimi date, an ethylene glycol characterized by formula ICH , OH-), wherein n is 2, 3, 4, 5, 6, 7, 8, 9, 10, 11, 12, 13, 14, 15, 16, 17, 18, 19, 20, 21, 22, 23, 24 or 25 and one or both termini of the ethylene glycol are substituted by a succinimide or maleimide group, N-(K-Maleimidound ecanoyloxy) sulfosuccinimide ester, sulfosuccinimidyl (4 - iodoacetyl) aminobenzoate, 1, 8-bismaleimidodiethyl eneglycol and 1, 11-bismaleimidotriethyleneglycol.

In a particular embodiment, the combination according to the present invention comprises:
- the anti-BAFF receptor antibody and the siRNA, and
- the anti-BCMA-specific receptor antibody and the siRNA,
antibody components of which are each conjugated with a small basic protein, preferably a protamine.

Synthesis techniques for the preparation of suitable peptide/protein-RNA linker molecules are conventional, and any of the techniques listed below may be employed in the context of the present invention, such as those described in WO2009083738, relevant portions incorporated herein by reference. Incorporation by reference herein is made to the following U.S. patents: U.S. Pat. Nos. 5, 138, 045 and 5,218,105, drawn to polyamine conjugated oligonucleotides; U.S. Pat. No. 5, 212, 295, drawn to monomers for the preparation of oligonucleotides having chiral phosphorus linkages; U.S. Pat. No.5,378,825 and U.S. Pat. No. 5,541,307, drawn to oligonucleotides having modified backbones; U.S. Pat. No. 5,386,023, drawn to backbone - modified oligonucleotides and the preparation thereof through reductive coupling ; U.S. Pat. No.5,457,191, drawn to modified nucleobases based on the 3 - deazapurine ring system and methods of synthesis thereof; U.S. Pat. No. 5,459,255, drawn to modified nucleobases based on N - 2 substituted purines; U.S. Pat. No. 5,521,302, drawn to processes for preparing oligonucleotides having chiral phosphorus linkages; U.S. Pat. No. 5,539,082, drawn to peptide nucleic acids, U.S. Pat. No. 5, 554, 746, drawn to oligonucleotides having lactam backbones; U.S. Pat. No. 5,571,902, drawn to methods and materials for the synthesis of oligonucleotides ; U.S. Pat. No. 5,578,718, drawn to nucleosides having alkylthio - groups , wherein such groups may be used as linkers to other moieties attached at any of a variety of positions of the nucleoside ; U.S. Pat. No. 5,587,361 and U.S. Pat. No. 5,599,797, drawn to oligonucleotides having phosphorothioate linkages of high chiral purity; U.S. Pat. No. 5,506,351, drawn to processes for the preparation of 2 - O - alkyl guanosine and related compounds, including 2,6-diaminopuhne compounds; U.S. Pat. No. 5,587,469, drawn to oligonucleotides having N - 2 substituted purines; U.S. Pat. No. 5,587,470, drawn to oligonucleotides having 3-deazapurines; U.S. Pat. No. 5,223,168, and U.S. Pat. No. 5,608,046, both drawn to conjugated 4'-desmethylnucleoside analogs; U.S. Pat. No. 5,602,240, and U.S. Pat. No. 5,610,289, drawn to backbone - modified oligonucleotide analogs; U.S. Pat. No. 6,262,241, and U.S. Pat. No. 5,459,255, drawn to, *inter alia*, methods of synthesizing 2-fluoro-oligonucleotides.

Many different linkers can be used with the present invention. In the present invention, linkers were used to associate a protamine or a small protein with BAFF receptor-specific mAb and with BCMA receptor-specific mAb. In one non-limiting example, a protamine linker (also referred to herein as a coupling reagent) can be used. A protamine linker generally comprises amino acids 8 - 29 of protamine, namely RSQSRSRYYRQRQRSRRRRRRS SEQ ID NO: 1. Other protamine sequences (e.g. a peptide comprising at least amino acids 12 - 20, at least amino acids 10 - 24, or at least amino acids 10 - 26 of protamine) are equally suitable. The linker can be incorporated at the Nor C- terminus of the translocation component, or within a surface exposed loop region of translocation component. In another example, a polylysine linker (also known as polyK linker) is used. One polyK linker comprises 5 - 30 or 5 - 20 or 5 - 15 or 7 - 10 lysine residues, optionally including one or more (but preferably no more than 5) non - lysine residues. The linker can be incorporated at the N - or C - terminus of the translocation component, or within a surface exposed loop region of translocation component. In another example, a TPTV linker (also known as a HIV - TaT protein translocation domain linker) can be used. One such TPTV linker comprises residues 47 - 57 of HIV TAT. TPTV linkers typically comprise 5-30 or 5-20 or 5-15 or 7-10 amino acid residues. The linker can be incorporated at the N- or C- terminus of the translocation component, or within a surface exposed loop region of translocation component.

Preferably, the antibody-siRNA complexes of the combination according to the present invention are PEGylated.

PEGylation is a biochemical modification process of bioactive molecules with polyethylene glycol (PEG), which lends several desirable properties to proteins/peptides, antibodies, and vesicles considered to be used for therapy or genetic modification of cells. PEGylation is the process of both covalent and non-covalent attachment or amalgamation of polyethylene glycol polymer chains to molecules and macrostructures, such as a drug, therapeutic protein or vesicle, which is then described as PEGylated.

Even more preferably, the disulfide bonds in the mAb are firstly reduced and secondly the antibody-RNA complexes of the combination according to the present invention are PEGylated.

The inventors observed that a partial reduction of disulfide bonds in mAb prior to PEG treatment of mAbs markedly improved the conjugate-mediated reduction of autoantibody levels, likely through the reduced formation of aggregates and enhanced intracellular uptake of the PEGylated conjugates, and greater suppression of critical receptors in consequence. Thus, they demonstrated that PEGylation further improves the efficacy of duoconjugate treatment in terms of a higher reduction in the level of autoantibodies. To resume, the attachment of PEG polymer to the antibody siRNA conjugate (PEGylated conjugate) further augment immunosuppressive effects.

The present invention also relates to a composition comprising the combination according to the invention.

Preferably, the composition according to the invention is adapted for intravenous, intramuscular, oral, parenteral, enteral, intraperitoneal, pulmonary, nasal, subcutaneous, rectal, or transcutaneous administration.

The present invention also relates to the composition according to the invention for use as a medicament. The medicament contains also a pharmaceutically acceptable medium.

A pharmaceutically acceptable medium includes any, and all solvents, dispersion media, coatings, antibacterial and antifungal agents, isotonic and absorption delaying agents and the like suitable for administration to a mammalian host. The use of such media and agents for pharmaceutical active substances is well known in the art. Supplementary active ingredients can also be incorporated into the medicament of the present invention.

The composition according to the invention is preferably used for the treatment of autoimmune disorders.

Autoimmune and more generally "Immunity" refers to the body's ability to defend itself against foreign substances, such as pathogenic microorganisms or cancer cells, by triggering an immune response. When a foreign body enters the body, the immune system is triggered by the production of messengers such as interleukins and cytokines.

The term "autoimmune disorder" as used herein, refers to a condition in which the body's immune system mistakes its own healthy tissues as foreign and attacks them. Most autoimmune diseases cause inflammation that can affect many parts of the body. The parts of the body affected depend on which autoimmune disease a person has. Common signs and symptoms include fatigue, fever, muscle aches, joint pain and swelling, skin problems, abdominal pain, digestion problems, and swollen glands. The symptoms often come and go and can be mild or severe. There are many different types of autoimmune diseases.

The composition for use according to the invention is preferably used for the treatment of autoimmune muscle disorders, more preferably *Myasthenia gravis.*

As used herein, the term *"myasthenia gravis"* or MG refers to any chronic progressive muscular weakness. Myasthenia gravis is an autoimmune disease that results from antibodies that block nicotinic acetylcholine receptors at the junction between the nerve and muscle, which prevents nerve impulses from triggering muscle contractions.

Patients with MG who are seropositive for autoantibodies to the acetylcholine receptor (AChR), muscle specific tyrosine (MuSK), or low-density lipoprotein receptor-related protein 4 (LRP4) present with voluntary muscle weakness due to dysfunctional neuromuscular junctions and impaired neuromuscular transmission. 70% of patients with MG exhibit seropositivity for AChR-specific autoantibodies and significantly reduced muscle function. The clinical signs of muscle fatigue include ptosis, diplopia, slurred speech, appendage tremor, and even respiratory failure due to disease worsening.

Preferably, in the composition for use according to the invention, the combination of the two antibody-RNA complexes is an immunosuppressant.

The term "immunosuppressant" or "immunosuppressive agents" or "immunosuppressive drugs", as used herein, refers to an agent that can suppress or prevent the immune response. Immunosuppressants are used to prevent rejection of a transplanted organ and to treat autoimmune diseases. Immunosuppressive drugs interfere at different stages and levels of the immune response.

As illustrated in the examples, the inventors were able to highlight that the combination of two conjugates, anti-BAFF receptor antibody siRNA and anti-BCMA receptor antibody siRNA produce synergistic/additive immunosuppressive effects. Indeed, it reduces the production of pathogenic antibody from B cells.

Preferably, the composition for use according to the invention is provided in a dose between 5 and 25 mg/kg of total mammal body weight.

In a more preferred embodiment, the composition is provided in a dose between 5 and 22 mg/kg of total mammal body weight.

In an even more preferred embodiment, the composition is provided in a dose between 5 and 20 mg/kg of total mammal body weight.

The invention also relates to composition for use according to the invention, wherein the composition comprises:
- an anti-BAFF receptor antibody or binding fragment thereof that is bound or conjugated to a small RNA that targets a BAFF receptor mRNA, and
- an anti-BCMA specific receptor antibody or binding fragment thereof that is bound or conjugated to a small RNA that targets a BCMA receptor mRNA,
as a combination of two antibody-RNA complexes product for a simultaneous, separated or time-separated administration.

Preferably, the composition for use according to the invention comprises:
- an anti-BAFF receptor antibody or binding fragment thereof that is electrostatically bound or conjugated to an siRNA, an shRNA, or both, that targets a BAFF receptor mRNA, and
- an anti-BCMA specific receptor antibody or binding fragment thereof that is electrostatically bound or conjugated to an siRNA, an shRNA, or both, that targets a BCMA receptor mRNA,
as a combination of two antibody-RNA complexes product for a simultaneous, separated or time-separated administration.

### EXAMPLES

Each experiment was repeated at least thrice. The data were compared and evaluated using a one-way analysis of variance, followed by Holm-Sidak or Tukey's post hoc analysis wherever applicable. Statistical significance was set at p < 0.05. The Biostatistics Core at UTMB further performed power determination analyses for each experiment. All statistical analyses assumed a 95% level of confidence.

### Example 1: Animals, EAMG Induction, and Clinical Evaluation

C57BL/6 mice (Jackson Lab, Bar Harbor, ME) were housed and maintained in a barrier facility at the University of Texas Medical Branch (UTMB), Galveston, Texas, following the guidelines of the National Institutes of Health and UTMB Animal Care and Use Committee. The UTMB Animal Care and Use Committee reviewed and approved this animal study.

MG-like disease (EAMG) is induced in mice through immunization with Torpedo AChR emulsified in complete Freund's adjuvant (CFA). The model typically presents clinical signs and characteristics similar to the MG features in humans, including high-affinity anti-AChR antibodies, immunoglobulin G (IgG), complement deposits at the neuromuscular junction, and reduced number of AChRs at postsynaptic membrane folds.

The affinity-purified Torpedo AChR was emulsified in CFA and used to immunize C57BL/6 mice (8 weeks, male) thrice.). MG disease in these mice was confirmed by an elevated anti-AChR antibody in serum. Clinical grades of the disease were determined through blinded observation (grade 0, normal muscle strength; grade 1, normal at rest but weak at exercise and hunchback posture with face down and reduced mobility; grade 2, grade 1 plus weakness at rest; and grade 3, moribund, dehydrated, and paralyzed). A digital recording of grip strength of the forelimbs (Dynamometer) was performed once at pretreatment and then during posttreatment weeks 2 and 6 and months 3 and 4.

### Example 2: Preparation and Verification of mAb-siRNA Conjugates

Both BR- and BCMA-specific siRNAs (Ambion In Vivo Pre-designed, HPLC purified; Genbank Accession Numbers NM_028075.2 and NM_011608.1) and in *vivo* negative control siRNA were purchased from ThermoFisher Scientific (Waltham, MA). BR siRNAs with 57.9% versus 36.8% GC content and BCMA siRNAs with 42% versus 33% of GC content were compared. Antimouse antibodies against BR (tumor necrosis factor receptor superfamily 13c) and BCMA (Tnfrsf17), the internalizing antibodies, were either purchased from LS Bioscience (Seattle, WA) or Adipogen (San Diego, CA) or were obtained from a custom-made hybridoma clones (RayBiotech, Peachtree Corners, GA). BCMA antibodies (Vicky-1) (ThermoFisher) were also assessed for cross-reactivity in mice and used in the experiments. All mAb and siRNA activities were validated through flow cytometry, quantitative reverse transcription-PCR (qRT-PCR), or Western blot using splenocytes and lymph node (LN) cells obtained from the EAMG mice.

BR- and BCMA-specific smt conjugates were prepared using protamine-a small, <7-kDa protein (Sigma, St. Louis, MO)-and heterobifunctional linkers (Trilink Biotech, San Diego, CA). In brief, buffer-exchanged mAb (3 mg/mL) and protamine (0.5 mg) were modified using Sulfo (S)-4FB and S-HyNic (Trilink Biotech) for 2 h. Next, both molecules were desalted, and contaminants were removed by using the Zeba column (Pierce Biotechnology, Rockford, IL). Modified mAb and protamine were then combined and incubated overnight at 4°C. Unbound protamine was removed by gel filtration. Protaminated mAb (**mt**) was then conjugated to siRNA (1:1 or 4:1) and separated using a concentrator column (Abcam, Waltham, MA). The mt was quantified with the BCA protein assay (Pierce) using purified mouse IgG as a standard and stored in phosphate buffer saline (PBS) at 4°C until further use. The conjugates (**smt**) with appropriate controls were electrophoresed using a native 10%-20% polyacrylamide gel. The presence of siRNA and mAb components in each conjugate was verified by staining the gel, first with ethidium bromide and then with coomassie blue. Protaminated mAb:siRNA ratio of 4:1 or 1:1 resulted in an equal yield of conjugates.

Zeta potential values of the conjugates (quantifying the surface charge) were measured in triplicate at 25°C by laser Doppler velocimetry using a Zetasizer 2000 (Malvern Instruments, Malvern, UK) following dilution of the samples to 10% in distilled water. Conjugates were also assessed for their stability by incubating aliquots of BR-BCMA conjugates with fresh mouse serum at a 1:1 ratio (10 µl each) at 4°C for varying periods up to 24 h; the complexes were then resolved via polyacrylamide gel electrophoresis (PAGE; 10% gel) to check for signs of degradation.

To target particular B-cell subsets, each fusion mAb-siRNA conjugate was constructed using protamine covalently attached with B cell-targeting mAbs at the Fc region through heterobifunctional crosslinkers. The protaminated mAb subsequently formed strong electrostatic bonds with BR- or BCMA-specific siRNAs. The data further revealed that gel filtration removed the contaminating free protamine from the protaminated mAbs (BR mt and BCMA mt), and both siRNA and mAb components of the conjugate appeared as the same band in a native 20% polyacrylamide gel stained with ethidium bromide and coomassie blue, respectively (Figure 1A).

Unconjugated siRNA alone did not show any protein bands in the coomassie-stained gel. The gel-eluted conjugate retained both Fab and Fc, as was evident in gradient SDS-PAGE performed under denatured/nondenatured conditions (Figure 1A). The zeta potential of the conjugate particles (smt) was approximately -22 mV, indicating moderate stability of the complex (Figure 1B). The results of incubation of the conjugates with serum at a 1:1 ratio indicated that the conjugate (mAb-siRNA) was fairly stable even after24 h (Figure 1C), and by this time, the conjugates were expected to reach the target organs and exert the desired effects.

### Example 3: In Vivo imaging of EAMG mice and localization of the conjugates

Whole-body IVIS spectrum fluorescence imaging was noninvasively performed to determine the distribution and persistence of conjugates in *vivo* in EAMG mice. EAMG mice, after the second booster immunization, were intraperitoneally administered with either PBS or the conjugates of antimouse BR mAb and Alexa Fluor 555-labeled nonspecific siRNA and antimouse BCMA mAb and Alexa Fluor 488-labeled nonspecific siRNA. mAb and siRNA were used at 100 µg and 25 µg, respectively, per mouse. A day before the treatment, mice were depilated in the abdomen with a commercial depilator (Nair, Church & Dwight). All mice were sedated with isoflurane and placed on an imaging shuttle of IVIS Spectrum In Vivo Imaging System (Perkin Elmer, MA). The shuttle was equipped with gas anesthesia connections for live imaging. Images were acquired by using excitation/emission wavelengths of 535 nm/580 nm to capture Alexa-555⁺ images and then 465 nm/520 nm to capture Alexa-488⁺ images 3 h after conjugate administration. Images were retaken after 24 h using the CCD camera of the IVIS equipment. Afterward, the mice were euthanized; inguinal LNs and kidneys were dissected out to directly compare the lymphoid and nonlymphoid organs for the fluorescence intensity of Alexa Fluor 488 and 555.

Earlier studies have shown that mAb-siRNA conjugates could internalize into transformed B-cells in *vitro* and primary B cells isolated from EAMG mice. To further explore the time-dependent biodistribution of B cell-targeting conjugates in *vivo,* EAMG mice were intraperitoneally injected with BR- and BCMA-specific mAbs conjugated to fluorescent siRNAs labeled with Alexa-555 (red/orange) and Alexa-488 (green), respectively. Both conjugates exhibited fluorescence in the vicinity of the intraperitoneal injection site 3 h after injection. After 24 h, the conjugate containing Alexa-555 was not detected at the injection site but was observed at the upper left of the abdomen, suggesting its likely accumulation in the spleen. Alexa 488 conjugate was detectable at 3 h but not at 24 h after injection, probably because of its weak fluorescence that was photobleached with time; therefore, the fluorescence was undetectable on the skin surface. No background fluorescence was detected in control mice. Inguinal LNs from PBS control- and fluorescent conjugate-treated mice were dissected out to observe the fluorescence in the LNs, which was not apparent on the mouse skin surface. EAMG mice injected with Alexa-555 conjugates exhibited a relatively higher fluorescence than autofluorescence in the LNs of PBS-treated mice; however, no fluorescence was detected in the kidneys, a nonlymphoid organ.

The results indicate that the B cell-targeting conjugates with particular affinity for B cells are preferentially directed toward those cells predominantly residing in the lymphoid organs.

### Example 4: Duoconjugates Reduced Target Receptor Expression

### Dose Optimization and Treatment

To optimize the dose of duo-conjugates, two weeks after the 2^{nd} boost, the EAMG mice with an established disease of grade 2 or 3 were randomized into various groups (n = 7). The mice were intraperitoneally treated once with BR- and BCMA-specific conjugates (smt) at a dose of 100, 125, or 150 µg. Some groups of mice were administered with protaminated mAbs (mt) or PBS to serve as controls. The treatment with BCMA-specific conjugates was initially performed 1 day later with BR-specific conjugates to avoid potential dose-overload. However, the mice could tolerate the subsequent simultaneous administration of duoconjugates with no signs of stress or adverse effects.

A separate set of EAMG mice groups (n = 5) was treated or pretreated once with an anti-interferon (IFN)-α/β receptor (IFNAR)-monoclonal antibody (MAR1-5A3) (Leinco Technologies, Fenton, MO) at a dose of 1-2.5 mg per mouse per manufacturer recommendation. Conjugates containing siRNAs with a high or low GC content (Assay IDs: s90521 [57.9%] and s90522 [36.8%] for BR and s75270 [42%] and s75269 [33%] for BCMA; ThermoFisher Scientific) were also administered to EAMG mice. To improve serum stability and intracellular uptake, conjugates were also PEGylated before treatment. In brief, MS(PEG) 12 (ThermoFisher Scientific) was dissolved in dimethylformamide and added to 2-mercapto-ethylamine (ThermoFisher Scientific)-treated mAbs in an amine-free buffer in the presence of the linker S-4FB. The mixture was incubated for 30 min at room temperature and then desalted. The PEGylated, linker-modified mAb was then incubated with linker-modified protamine and subsequently reacted with siRNA to obtain PEGylated mAb-siRNA conjugates. To verify if non-targeting siRNA nonspecifically reduces BR or BCMA transcripts, EAMG mice were treated with conjugates containing either BR or BCMA mt with nonspecific siRNAs. All conjugates were quantitated, diluted in PBS, and intraperitoneally injected into mice.

All mice were euthanized 3 days posttreatment (dpt), 6 weeks posttreatment (wpt), and 3 and 4 months posttreatment (mpt) for different sets of the experiment.

### Cell Preparation and Flow Cytometry Analysis

Mice were bled from the tail vein to isolate peripheral blood mononuclear cells (PBMCs) and collect serum. The mice were euthanized at various time points (dpt/wpt/mpt) to remove tissues and isolate splenocytes and LN cells (inguinal and brachial). PBMCs or splenocytes (>4 × 106 cells) were processed using Lymphoprep on SepMate tubes (StemCell Technologies, Cambridge, MA). Cells were treated with the red blood cell (RBC) lysis solution (Sigma) to remove contaminating RBCs. The cells were then subjected to flow cytometry and qRT-PCR. Specimen tissues were snap-frozen in pieces for qRT-PCR and other downstream analyses.

All single-cell preparations (RBC-free) were washed with PBS-1% heat-inactivated fetal bovine serum. The cells were subjected (or not) to Fc receptor blocking and stained with the fluorochrome (e.g., FITC, PE, Alexa, APC, and PE-Cy7)-conjugated mAbs and secondary antibodies (as needed). Cells were either analyzed immediately or fixed in 1% paraformaldehyde. All cells were analyzed with a BD LSR Fortessa flow cytometer using the FACSDiva software (BD Biosciences, San Jose, CA) at the UTMB Flow Cytometry and Cell Sorting Core.

### qRT-PCR

Total RNA was isolated from PBMCs and pieces of the snap-frozen spleen and LN (inguinal) using TRIzol (Invitrogen, Carlsbad, CA). Genomic DNA-free complementary DNA was prepared from RNA using Superscript II, random primers, nucleotide mix, DNAse, and appropriate buffers (Invitrogen). Relative expression of BR, BCMA, B220, and beta-actin (for normalization) was analyzed using FAM-conjugated gene-specific probe-primer mix and AmpliTaq Gold TaqMan PCR master mix (Invitrogen) on a CFX 96 Real-Time system (Bio-Rad, Hercules, CA). Relative fold changes in mRNA, compared with those of the beta-actin mRNA (standard), were computed (2^{-ΔΔCt} method) using CFX software (Bio-Rad).

### Results

It was previously reported that a paradoxical increase in autoantibody levels (despite a slight reduction in B-cell frequencies) is associated with a high expression of type 1 IFN after treatment with the conjugates at a high dose (>350 µg, nearly 20 µg/g body weight). Therefore, dose optimization of the conjugates was performed by assessing reduced doses of ≤50% to induce the maximum inhibition of target receptors without triggering any interferonogenic or humoral response. Moreover, duoconjugates-BCMA-specific conjugates were used in addition to BR-specific conjugates-as BCMA is a highly specific receptor of plasma cells that produce chronic MG-specific autoantibodies. Thus, the goal was to maximally suppress the expression of target mRNA and B-cell receptors in both precursors and terminally differentiated plasma B cells that coordinate to exert stronger autoantibody-reducing effects. The study included four sets of experiments, as outlined in Table 1.

**Table 1. Study design.**

| Sets of experiments | EAMG | Treatment | Bleeding and euthanasia | Aims |
|---|---|---|---|---|
| 1: Dose optimization | Day 0 | Day 1 | Day 3 | Target receptor expression |
| 2: Effect of IFNAR Ab, differential GCs in siRNA and AChR-specific B cell analyses | Day 0 | Day 1 | Week 6 | Target receptor expression, serum autoAb levels |
| 3: Memory B cell frequencies | Day 0 | Day 1 | Month 3 | CD27 expression |
| 4: Treatment outcome | Day 0 | Day 1 | Week 6, Month 4 | Serum autoAb levels, grip strengths and functional AChR |

To confirm the suppressive effects of the conjugates, the frequency of BR- and BCMA -expressing B cells in the spleen 3 dpt was assessed as a measure of the reduction in the number of mature and plasma B cells. Flow cytometry revealed that all three doses of the duoconjugates (100, 125, and 150 µg) substantially reduced BR- and BCMA-expressing and total B220⁺ splenocytes (20%-60%), significantly compared with PBS, mAb alone, or monoconjugates treatments (Figure 2A, a-c). Of note, owing to plasma cell-specific surface expression, BCMA-specific antibody treatment was not expected to significantly reduce BR⁺ cells (Figure 2A, a). Similarly, BR-specific antibody treatment was not supposed to affect BCMA⁺ B-cell frequencies; nevertheless, a slight reduction in the proportion of both cell types caused by both antibodies (Figure 2A, a and b) was found. The low frequencies of BCMA⁺ cells are likely because of an insufficient amount of antibodies, as a higher level of antibodies yielded BCMA⁺ cells in higher frequencies in a separate experiment (Figure 2B).

Because CD3⁺T cells also express low levels of BR, an examination of how CD3 cell frequencies were affected by the conjugate treatment was done. Only BR mt significantly reduced the frequency of these cells. In contrast, the BR-specific conjugate significantly increased the number of CD3⁺ T cells (Figure 2A, d).

Flow cytometry revealed a higher efficacy of duoconjugates in reducing BCMA⁺ B cells in the spleen than in PBMCs or the cells in LNs (Figure 2B, upper and lower panels). Consistent with the flow cytometry results, the qRT-PCR results further showed reduced mRNA levels of BR and BCMA (up to negative six-fold reductions) corresponding to the three doses of the duoconjugate treatment (Figure 2C).

In a follow-up repeat experiment, further dose escalation of mAb and siRNA, without overhauling the EAMG mice with high- or overdose-induced adverse effects, was determined to be nearly 200 µg-10 µg of protaminated mAb and 4 µg of siRNA per g of mouse body weight. A higher dose appears to be ineffective for a higher suppression of targeted receptors or reduction of the targeted subsets of B cells; a higher dose also exerts an opposing effect.

To conclude, the results show that a single treatment of EAMG mice with the optimized doses of conjugates, particularly duoconjugates and duo mAbs, considerably suppresses the crucial receptors, BR and BCMA, in targeted B cells, both at mRNA and protein levels.

### Example 5: Neutralization of Type 1 IFN and Effects of GC Content in siRNA component of the conjugate and AChR-Specific B Cells

A nonspecific induction of type 1 IFN by high-dose BR-specific monoconjugates despite the use of a modified siRNA to prevent the triggering of innate immune responses was previously reported. Type 1 IFN likely interfered with the autoantibody-reducing effects of the conjugates as it is known to promote antigen processing. To mitigate such plausible effects of IFN-α/β, an assessment of if antibody-mediated prior neutralization of IFN- α/β receptor (IFNAR) increases the efficacy of conjugate treatment by further reducing the frequencies of target cells and, thus, the levels of autoantibodies was done. Pretreatment with anti-IFNAR mAb at 2 mg per mouse followed by duoconjugate treatment slightly reduced CD19⁺ cell frequency at 6 wpt (Figure 3A). However, solo treatment with IFNAR antibody (without conjugates) resulted in a paradoxical effect (increased surface expression of target receptors; Figure 3A). The data also show that Fc-blocked CD19⁺ B-cell numbers (representing the same BR-positive transitional/mature B-cell subset) were substantially lower in the LN at 6 wpt. Owing to the plasma cell-specific expression of BCMA, the BCMA-only conjugate did not reduce the number of CD19⁺ transitional B cells (Figure 3A).

To resume, the suppressive effect of BR and BCMA receptors led to a modest reduction in the number of B cells (CD19 and B220) for at least 6 wpt.

Next, the effect of GC content of the siRNA component of the conjugates on B cell receptor suppression was compared. GC content of siRNA reportedly affects its activities, such as a high GC content may confer stability to the siRNA through secondary structures. In contrast, a low GC content lacks this property but facilitates the binding of siRNA with the target mRNA. To elucidate if differential GC contents of siRNA further reduce the expression of surface receptors through increased mRNA degradation, 21-mer siRNAs that contained approximately 20% greater or lower GC content was used. A higher GC content of siRNA showed efficacy in reducing CD19⁺ B cells compared with a low GC content; however, the differences were not statistically significant among the treatment groups (Figure 3B).

Previously a positive correlation of autoantibody levels with the AChR-specific B cells that are detectable using fluorochrome Alexa 647-conjugated to Torpedo AChR has been shown. A correlation between the altered GC content (high vs. low) and the increased apoptosis of AChR antibody-producing and Fas receptor (CD95)-coexpressing B cells was detected. A low GC content of the conjugate correlated with relatively lower expression of Fas receptor in LN cells; however, the differences in AChR⁺ CD95⁺ cell frequencies between the high and low GC content of the siRNA-containing conjugates were not statistically significant (Figure 3C).

### Example 6: CD27⁺ Plasmablasts

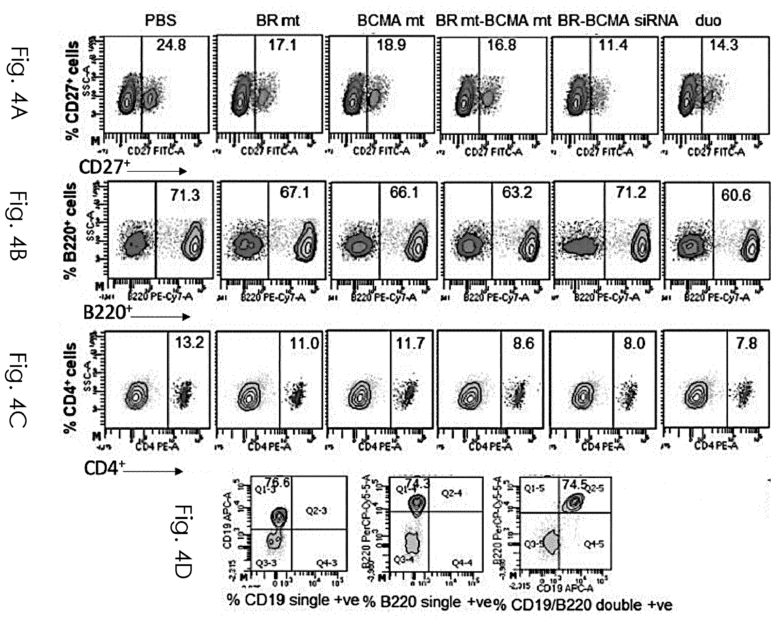

Splenic B cells from a separate set of mice 3 mpt were purified to assess the generation of total spleen-resident memory B or T cells. The frequencies of total CD27⁺ cells were determined, representing the population of splenic plasmablasts. There are conflicting reports on CD27⁺ cells coexpressing B220 or CD19. The results showed that the proportion of total splenic CD27⁺ cells, but not that of B220⁺ cells, was considerably reduced 3 months after the duoconjugate treatment (Figure 4). Deficiency in the proportion of the B220⁺ subset by conjugate treatment appears to have been gradually restored within 3 mpt. The CD4⁺ cell numbers were also slightly reduced 3 mpt (Figure 4C) due to the presence of BR on these cells.

To conclude, the number of splenic CD27+ total memory B/T cells (also representing plasmablasts) significantly declined within 3mpt, suggesting an association of reduced memory B cell frequencies with impaired autoantibody function.

### Example 7: AChR-Specific Autoantibody Levels

Anti AChR-antibody levels were measured by ELISA by using a pre-titrated dilution of serum and an affinity-purified mouse muscle AChR as the coating antigen, horseradish peroxidase (HRP)-conjugated rat antimouse IgG2b as the secondary antibody (Caltag, Burlingame, CA, California States and BD Biosciences), and 2'azino-bis (3-ethylbenzthiazoline-6-sulfonic acid) or 3,3',5,5'-tetramethylbenzidine (ThermoFisher) as substrates.

A highly specific nonradioactive method was developed to accurately measure relative serum levels of autoantibodies in samples using biotinylated α-bungarotoxin (BTX) and magnetic streptavidin beads. For the antibody assay, serum anti-AChR IgG/IgG2b was immunoprecipitated using biotinylated BTX (ThermoFisher Scientific) and affinity-purified AChR in Tris-EDTA-Tween 20 buffer and incubated overnight on a rotating platform with magnetic streptavidin (Cell Signaling Technology, Danvers, MA). Pulled-down complexes in beads were washed, mixed with a loading dye, and then resolved in sodium dodecyl sulfate (SDS)-PAGE (4%-20%; Bio-Rad). The protein was transferred onto a polyvinylidene fluoride membrane blocked with 5% nonfat dry milk and probed with an HRP-conjugated goat antimouse IgG2b (Santa Cruz Biotechnology, Santa Cruz, CA and Caltag) at 2:5000 dilution in Tris Buffer Saline-Tween 20 (0.05%) for 1 h at room temperature. Enhanced chemiluminescence (Pierce Biotechnology) assay was performed, and the gel image was captured using a GE Amersham Imager 680. The image revealed a band representative of the anti-AChR IgG2b level in each sample.

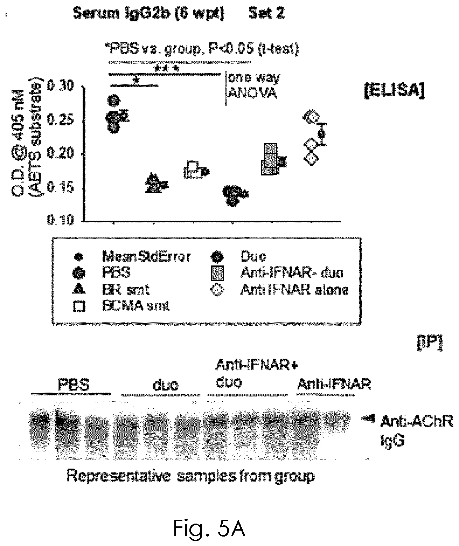

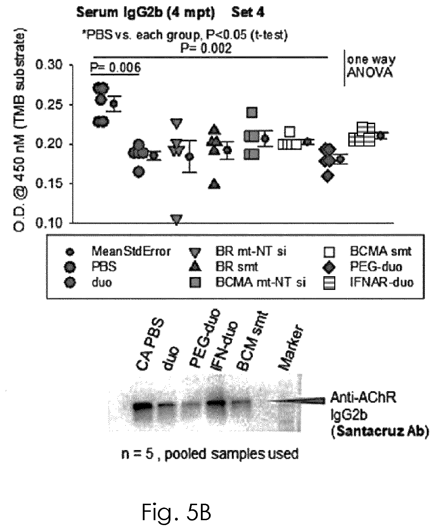

To assess the extent to which BR-BCMA-specific conjugates reduced the level of autoantibodies and the duration of the persistence of reduced autoantibodies, AChR-specific antibodies in EAMG mice in different sets of experiments were evaluated throughout the study period. Pathogenic anti-AChR IgG/IgG2b levels were measured by ELISA and a custom-developed antibody precipitation assay (more specific than ELISA). High-affinity AChR-specific antibodies were detected in the serum of PBS-treated EAMG mice compared to the serum of mice receiving the other treatments. At any posttreatment time point assessed, duoconjugate and BR-specific monoconjugate treatment markedly reduced the level of anti-AChR antibodies (Figures 5A and 5B). PEGylation of duoconjugates effectively lowered autoantibody levels more than the non-PEGylated duoconjugates. At 4 mpt, only duoconjugate and PEGylated duoconjugate treatments could still significantly reduce the level of autoantibodies. Pretreatment of EAMG mice with anti-IFNAR mAb reduced the autoantibody levels compared with EAMG mice only. However, the pretreatment, even with a higher dose, showed no efficacy at any time to further reduce the level of conjugate-mediated reduction of autoantibodies.

Treatment-mediated deficiency of B220+ B cells was restored substantially within 3 mpt, but the reduction in autoantibody levels persisted from 6 wpt to 3 mpt and beyond until the experimental endpoint of 4 mpt, found in different sets of experiments.

### Example 8: Clinical Grade of EAMG Mice

At 6 mpt, 70% of the EAMG mice treated with either mono- or duoconjugates showed grade-0 disease with normal mobility, and 30% had a clinical grade between 1 and 2. All EAMG mice treated with PBS developed the grade-3 disease. PEGylated conjugates or anti-IFNAR mAb-treated mice developed the grade-2 disease. Differences in the body weight among the various groups of mice were not statistically significant.

### Example 9: Grip Strength of EAMG Mice

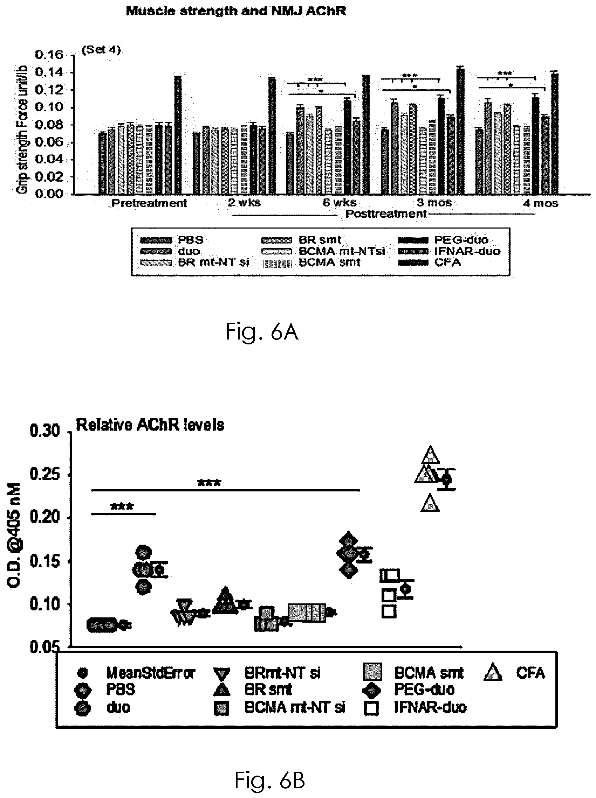

A digital recording (Dynamometer) of the forelimb grip strength in EAMG mice revealed considerable improvements in muscle strength at week 6 and month 4 posttreatment (but not earlier than weeks 2 or 4) when compared to the PBS-treated control groups. Body weights of the mice did not vary significantly at any experimental timepoint (Figure 6A).

### Example 10: Functional AChR Levels in the Muscle

For functional AChR analysis, crude AChRs were obtained from the membrane fraction derived from homogenized mouse forelimb muscle (proximal) tissues. ELISA was performed following the method described previously.

Forelimb muscle samples obtained from the EAMG mice that were untreated or treated with PBS or BR--BCMA conjugates or left untreated were quantitated by the ELISA method, as described. Muscle AChR levels were significantly preserved only with the duo- and PEGylated duoconjugate treatments (Figure 6B).

The clinical improvements such as disease grade, grip strength, and functional muscle AChR level resulting from treatment with the duoconjugates (BR and BCMA) and PEGylated duoconjugates were improved to a greater extent and significantly than those from monoconjugate treatment.

## Claims

1. A combination of two antibody-RNA complexes comprising:
- an anti-BAFF receptor antibody or binding fragment thereof that is bound or conjugated to a small RNA that targets a BAFF receptor mRNA, and
- an anti-BCMA specific receptor antibody or binding fragment thereof that is bound or conjugated to a small RNA that targets a BCMA receptor mRNA.

2. The combination according to claim 1, wherein said small RNA are miRNA, shRNA, siRNA, antisense RNA, and/or specific mRNA-targeting base/nucleotide sequence(s) preferably siRNA.

3. The combination according to claim 2, wherein:
- the anti-BAFF receptor antibody and the siRNA, and
- the anti-BCMA-specific receptor antibody and the siRNA,
in which antibody and siRNA are electrostatically bound via small protein chemically cross-linked to the antibody with a linker.

4. The combination according to claim 3, wherein the linker is a conditionally self-cleaving RNA sequence, a pH sensitive linker, a hydrophobic sensitive linker, a cleavable linker, a linker that provides a sorting signal, a linker that reduces steric hindrance, a linker that contributes to a condensing ability of the nucleic acid binding domain, a peptide or protein linker, a protamine linker, a polyK linker, or an HIV-TaT protein translocation ( TPTV ) linker.

5. The combination according to claim 3, wherein the linker is selected from glutaraldehyde, bissul fosuccinimidyl suberate, carbodiimide, bis ( succinimidyl ) penta ( ethylene glycol ), bis ( succinimidyl ) nona ( ethylene glycol ), bis ( sulfosuccinimidyl ) suberate, dimethyl suberimi date, an ethylene glycol **characterized by** formula ICH , OH -), wherein n is 2, 3, 4, 5, 6, 7, 8, 9, 10, 11, 12, 13, 14, 15, 16, 17, 18, 19, 20, 21, 22, 23, 24 or 25 and one or both termini of the ethylene glycol are substituted by a succinimide or maleimide group, N-(K- Maleimidound ecanoyloxy) sulfosuccinimide ester, sulfosuccinimidyl (4 - iodoacetyl) aminobenzoate, 1,8- bismaleimidodiethyl eneglycol and 1, 11- bismaleimidotriethyleneglycol.

6. The combination according to claims 1 and 2, wherein:
- the anti-BAFF receptor antibody and the siRNA, and
- the anti-BCMA-specific receptor antibody and the siRNA,
antibody components of which are each conjugated with a small basic protein, preferably a protamine.

7. The combination according to one of the preceding claims, wherein the antibody-siRNA complexes are PEGylated.

8. A composition comprising the combination according to one of the preceding claims.

9. The composition according to claim 8, wherein it is adapted for intravenous, intramuscular, oral, parenteral, enteral, intraperitoneal, pulmonary, nasal, subcutaneous, rectal, or transcutaneous administration.

10. The composition according to claim 8 or 9, for use as a medicament.

11. The composition for use according to claim 10, for the treatment of autoimmune disorders.

12. The composition for use according to claim 11, for the treatment of autoimmune muscle disorders, preferably *Myasthenia gravis.*

13. The composition for use according to claim 12, wherein the combination of the two antibody-RNA complexes is an immunosuppressant.

14. The composition for use according to claims 10 to 13, wherein the composition is provided in a dose between 5 and 25 mg/kg of total mammal body weight.

15. The composition for use according to claims 10 to 14, wherein the composition comprises:
- an anti-BAFF receptor antibody or binding fragment thereof that is electrostatically bound or conjugated to a small RNA that targets a BAFF receptor mRNA, and
- an anti-BCMA specific receptor antibody or binding fragment thereof that is electrostatically bound or conjugated to a small RNA that targets a BCMA receptor mRNA,
as a combination of two antibody-RNA complexes product for a simultaneous, separated or time-separated administration.
